# EUROPEAN PATENT SPECIFICATION

(11) **EP 1 610 796 B1**
(45) Date of publication and mention of the grant of the patent: **23.04.2014**
(21) Application number: 04757552.7
(22) Date of filing: 17.03.2004
(51) Int. Cl.: A61K 31/485, A61P 25/00

(54) **HIGH POTENCY DOPAMINERGIC TREATMENT OF NEUROLOGICAL IMPAIRMENT ASSOCIATED WITH BRAIN INJURY**
HOCHPOTENTE DOPAMINERGE BEHANDLUNG VON NEUROLOGISCHEN BEEINTRÄCHTIGUNGEN IN VERBINDUNG MIT HIRNVERLETZUNGEN
TRAITEMENT DOPAMINERGIQUE TRES PUISSANT DE DEFICIENCES NEUROLOGIQUES ASSOCIEES A DES LESIONS DU CERVEAU

(30) Priority: 17.03.2003 US 455405 P
(43) Date of publication of application: 04.01.2006
(73) Proprietor: Neurohealing Pharmaceuticals, Inc., Brookline, MA 02445 (US)
(72) Inventor: KATZMAN, Daniel, E., Brookline, MA 02445 (US); GAMZU, Elkan, R., Newton, MA 02159 (US); FARBER, Neal, M., Waban, MA 02468 (US); FRIDMAN, Esteban, A., Capital Federal (AR); MERELLO, Marcelo, Capital Federal (AR)
(74) Representative: Brady, Paul Andrew
(86) International application number: PCT/US2004/008120
(87) International publication number: WO 2004/082630

(56) References cited:
- US-A- 3 961 060
- US-B1- 6 310 085
- DATABASE EMBASE [Online] ELSEVIER SCIENCE PUBLISHERS, AMSTERDAM, NL; 1952, VELLUZ J: "Barbituric coma" XP002502435 Database accession no. EMB-0007176402 & REVUE DU CORPS DE SANTE MILITAIRE 1952, vol. 8, no. 2, 1952, pages 275-282, ISSN: 0370-4173
- TANG ET AL.: "Involvement of activation of dopaminergic neuronal system in learning and memory deficits associated with experimental mild traumatic brain injury" EUROPEAN JOURNAL OF NEUROSCIENCE, vol. 9, 1997, pages 1720-1727, XP002500947
- MITCHELL ET AL.: "Coma arousal procedure: a therapeutic intervention in the treatment of head injury" BRAIN INJURY, TAYLOR AND FRANCIS, LONDON, GB, vol. 4, no. 3, 1 January 1990 (1990-01-01), pages 273-279, XP009107537 ISSN: 0269-9052
- HAIG A J ET AL: "Recovery from vegetative state of six months' duration associated with sinemet (Levodopa/Carbidopa)" ARCHIVES OF PHYSICAL MEDICINE AND REHABILITATION, W.B. SAUNDERS, vol. 71, 1 January 1990 (1990-01-01), pages 1081-1083, XP009107478 ISSN: 0003-9993
- SPIRIDONOV V.K. ET AL: 'Activity of dopaminergic endings of rat forebrain nuclei during electroconvulsive amnesia and restoration of conditioned reaction of passive avoidance with apomorphine' JOURNAL VICSEJ NERBNOJ DEJATELNOCTI vol. 31, no. 2, 1981, pages 409 - 411, XP002982377

## Description

### Cross-Reference to Related Applications

This application claims priority to U.S. Provisional Application No. 60/455,405, filed March 17, 2003.

### Field of the Invention

This invention is related to the fields of neurology and neurorehabilitation. In particular, this invention is related to treatments to restore impaired neurological function associated with brain injury in an individual.

### Background of the Invention

There are approximately 1.5 million new cases of head injuries to people in the United States every year. Of these, about 300,000 are severe enough to require hospitalization, and 50,000 to 75,000 will result in a coma that lasts for more than two weeks. Due to constant advances in effective emergency medicine, many patients that sustain a brain injury due to any of a variety of trauma will survive but remain in a severe altered state of consciousness, such as a coma, the deepest state of unconsciousness, or another more emergent but altered state of consciousness, such as persistent vegetative state (PVS) or minimally conscious state (MCS). Such patients fail to emerge to the fully, functional state of awareness of self and environment that they possessed prior to brain injury. Occasionally, some patients may emerge from a particular, deeper state of altered consciousness to a higher state, or even to normal awareness. However, even with emergence to full awareness, it is not uncommon for such individuals to require some form of neurorehabilitation to improve or regain any of a number of neurological functions, such as communication skills, motor skills, memory skills, and various other cognitive functions that permit self care, mobility, and employability. Clearly, patients that are in or have emerged from a severely altered state of consciousness represent a significant emotional, social, and economic burden to their families and to society.

Different therapeutic interventions have been proposed to aid the functional recovery of post-traumatic coma patients, but the results have been inconclusive. There have been a few case reports describing the use of different pharmacological agents on post-traumatic coma patients, e.g., using oral formulations of levodopa and carbidopa (Haig et al., Arch. Phys. Med. Rehabil., 71: 1081-1083 (1990)), bromocriptine (Passler et al., Arch. Phys. Med. Rehab., 82: 311-315 (2001)), methylphenidate (Whyte et al., J. Head Trauma Rehabil., 17(4): 284-299 (2002)), and amantidine (Wolf and Gleckman, Brain Injury, 9(5): 487-493 (1995); Meythaler et al., J. Head Trauma Rehabil., 17(1): 300-314 (2002)). The need for innovative clinical research on coma and other altered states of consciousness disorders has never been greater, since relatively recent advances in medicine have enabled survival of a higher percentage of patients of head injury. Yet, there are no generally accepted therapeutic options to promote emergence from an altered state of consciousness or to stimulate neurorehabilitation of patients of brain injuries. In Tang, Y.P. et al, Eur. J Neurosci, 9: 1720-1727 (1997) the use of apomorphine injected intraperitoneally in mice having had traumatic brain injury is described. That study concludes that apomorphine does not have any improving effect on the learning or memory deficits of the mice studied.

It has been suggested that coma duration is directly related to functional recovery as measured by any of a variety of disability scales used to assess patients, i.e., the longer a patient remains in an altered state of consciousness, the longer the patient requires some form of neurorehabilitative treatment. Such neurorehabilitative treatments may not only have the goal of restoring a patient with brain injury to a higher, preferably normal state of consciousness but also to restore or improve any of a variety of neurological functions that may have been impaired due to the brain injury, such as, communication skills (speaking, writing), cognitive skills (e.g., reasoning, memory), and motor skills (directed movements, walking, running, balancing). In addition, in recent years, important new findings have been made that indicate an ability of the neural network of the brain of a trauma patient to reorganize itself, a mechanism known variously as "neural plasticity", "axonal plasticity", "adaptive plasticity", or "activity-dependent plasticity", in which interactions between surviving neurons may adopt a new function or be recruited to restore a lost neurological function. A treatment that promotes a patient's emergence to a greater state of awareness should, in theory, also expedite the neurorehabilitation process. Clearly, needs remain for effective therapies to treat impaired neurological function in patients that have suffered brain injury.

### Summary of the Invention

The invention described herein addresses the above needs and problems by providing methods and means for treating impaired neurological function in individuals who have sustained a traumatic brain injury, comprising administering to the individual the high potency dopaminergic agent apomorphine. Impaired neurological functions treated according to the invention are the result of an injury to the brain, such as may arise from traumatic brain injury (e.g., resulting from a fall on a hard surface or vehicle accident, a strike to the head). Compositions for use in the treatment of an ischemic event (e.g., stroke), anoxic event, hypoxic event, a drug-induced injury (e.g., anesthesia-induced, drug overdose, illicit drug use), or as a result of major organ failure are also described herein.

Impaired neurological function associated with brain injury that may be treated with methods and compositions according to the invention include, but are not limited to, a less than normal state of consciousness (e.g., coma, near-coma, vegetative state, persistent vegetative state, minimally conscious state) and/or other impaired functions that are primarily cognitive functions (e.g., in addition to state of consciousness, memory, voice recognition), primarily sensory functions (e.g., tactile sensing, hot-cold sensing, light sensing), primarily motor functions (e.g., directed body movements, walking, maintaining balance), or a combination of (complex or integrated) neurological functions (e.g., speaking, writing, use of tools, operating machines).

The invention provides use of the highly potent dopaminergic agent apomorphine for treating one or more impaired neurological functions associated with a traumatic brain injury in an individual.

In a preferred embodiment, apomorphine is administered to an individual according to the invention at a dose in the range of from 30 to 200 mg of apomorphine per day (mg/day), more preferably, 48-128 mg/day. Daily dosing may be accomplished by single, multiple, or continuous injection or infusion of apomorphine into an individual. A preferred regimen for administering apomorphine to an individual according to the invention is to administer apomorphine at a rate of from 4 to 8 mg per hour for 12 to 16 hours per day.

Preferably, treatments according to the invention are applied to an individual for no longer than 18 to 24 months; more preferably, no longer than 12 to 18 months; more preferably, no longer than 6 to 12 months; most preferably no longer than 6 to 26 weeks. Treatments described herein may be applied to an individual more than once, e.g., after a pause or hiatus.

The invention may also comprise administering apomorphine to an individual in conjunction (e. g., co-administration, concurrent administration, sequential administration) with one or more other compounds that may provide additional therapeutic benefits, including, without limitation, anti-emetic agents and/or other dopaminergic agents. It is understood that various compounds may be administered to an individual in a mixture with apomorphine or separately, at the same time or different times (e.g., sequentially) as administering apomorphine, and/or by the same or different route as used for administering apomorphine.

Preferred anti-emetic agents useful in the invention include, but are not limited to, peripheral dopamine antagonists, phenothiazine agents, benzamide agents, serotonin antagonists, histamine antagonists (antihistamines), parasympathetic depressants, and meclizine agents. Other antiemetic agents that can be used in accordance with the present invention include metoclopramide; phenothiazines such as chlorpromazine, prochlorperazine, pipamazine, thiethylperazine and oxypendyl hydrochloride; serotonin (5-hydroxytryptamine or 5-IIT) agonists such as domperidone, odansetron,; histamine antagonists including buclizine hydrochloride, cyclizine hydrochloride, and dimenhydrinate; parasympathetic depressants such as scopolamine; metopimazine; trimethobenzamide; benzquinamine hydrochloride; and diphenidol hydrochloride. A particularly useful anti-emetic agent is domperidone. An anti-emetic agent is preferably, although not necessarily, administered to an individual prior to administration of apomorphine.

Apomorphine may be administered in combination with other dopaminergic agents according to this invention. Any of a variety of dopaminergic agents may be administered to an individual with apomorphine according to the invention including, but not limited to, L-dopa (levodopa), bromocriptine, amantadine, pergolide, pramipexole, ropinirole, fenoldopam, cabergoline, rotigotine, lysuride, talipexale, 7-OH DPAT, quinpirole, SKF-38393, and combinations thereof. When apomorphine is administered to an individual in conjunction with L-dopa according to the invention, the concentration of L-dopa may be similar to or less than the relatively high concentrations (e.g., 1000 mg or more per day) that are disclosed herein, which are those that employ L-dopa as the primary or only high potency dopaminergic agent to treat impaired neurological function(s) associated with a brain injury in an individual (see, below).

In still another embodiment of the invention, apomorphine may be administered to an individual for use in treating an impaired neurological function(s) in conjunction with any of a variety of neurorehabilitation programs for restoring neurological function. Neurorehabilitation programs useful in the invention include, without limitation, physical/sensory type protocols (exercises, tasks, light stimulation, voice stimulation, pictures, tactile stimulation), electric and/or magnetic stimulation regimens (e.g., trans-cranial magnetic stimulation (TMS), deep brain stimulation (DBS)), drug-based stimulation regimens (e.g., using modafinil, caffeine, amphetamines), and combinations thereof.

In another embodiment of the invention, apomorphine is administered to an individual parenterally (i.e., by a route outside the alimentary canal), including, without limitation, subcutaneously (s.c.), intravenously (i.v.), intramuscularly (i.m.), intra-arteriorally (i.a.). In a preferred embodiment, apomorphine is formulated for and administered subcutaneously for use according to the invention. Apomorphine may be advantageously administered manually or automatically via a medical device, such as a pump.

preferred dosage range for administering L-dopa to an individual according to the invention is from 1250 to 2500 mg/day.

The compositions for use in the invention may be used to treat impaired neurological function associated with a traumatic brain injury as manifested in an individual as any of a variety of altered consciousness state (ACS) disorders including, without limitation, coma, near-coma, vegetative state, persistent vegetative state, minimally conscious state, and the like. Such disorders are readily diagnosed and assessed using standard protocols of clinical neurology including, but not limited to, the Glasgow Outcome Scale, the Extended Glasgow Outcome Scale (GOS-E), the Kennedy Johnson Scale, the Disability Rating Scale, the Coma-Near Coma Scale, the Ranchos Amigos Scale, as well as standard neurological examination procedures that provide clinical impressions of change (CIC) in neurological function and, even, combinations thereof.

### Detailed Description

This invention provides compositions for use in treating impaired, i.e., diminished or lost, neurological function(s) in individuals who have sustained a traumatic brain injury. In particular, the invention is based on the discovery that use of the highly potent dopaminergic agent apomorphine, as described herein, is particularly effective at treating impaired neurological function associated with a brain injury in an individual. Dopamine receptors are involved in neural transmission for a variety of neurological functions, including those functions that are commonly characterized neurologically as cognitive functions, motor functions, or as a combination of cognitive and motor functions. Apomorphine directly binds dopamine receptors to stimulate neural transmission, whereas L-dopa is metabolized to dopamine, which is the naturally occurring neural transmitter that binds dopamine receptors to stimulate neural transmission in the dopaminergic system.

In order that the invention may be more clearly understood, the following terms and abbreviations are used as defined below.

The term "brain injury" is a general term used to refer to a condition that results in central nervous system damage, irrespective of the physiopathological source. Among the most frequent origins of a "brain injury" are stroke and traumatic brain injury (TBI).

A "drug" refers to any compound or composition that has a pharmacological activity. Thus, a "therapeutic drug" is a compound or composition that can be administered to an individual to provide a desired pharmacological activity. For dopaminergic agents, such as apomorphine, as described herein, the desired pharmacological activity is stimulation of dopaminergic neural transmission to treat an undesired or harmful disorder or condition associated with brain injury in an individual. Such disorders or conditions include, but are not limited to, altered states of consciousness and/or other neurological impairments. A "prophylactic drug" is a compound or composition that can be administered to an individual to prevent or provide protection from the development in an individual of an undesired or harmful disorder or condition. A drug may have prophylactic as well as therapeutic uses. An "illicit drug" refers to a drug that is generally illegal to possess and/or use under any circumstances in a particular jurisdiction without governmental authority. Illicit drugs include illegal "recreational" and "addictive" compounds and controlled substances such as various opiates and various psychotropic substances.

"Neurological function" refers to a function of the body of an individual that requires normal functioning neural transmission. Neurological functions of an individual that may be impaired by brain injury, and that are therefore treatable according to this invention, include, without limitation, functions that are primarily sensory (e.g., light sensing, tactile sensing, hot-cold sensing), primarily cognitive (e.g., state of consciousness, memory, comprehension, reasoning), functions that are primarily based on motor activity (e.g., directed body movements, walking, maintaining balance), or a combination of (i.e., complex or integrated) neurological functions (e.g., speaking, writing, use of tools, operating machines). Impaired neurological functions may also be referred to by the name for the corresponding neurological disorder, e.g., "amnesia" for loss of memory; a specific disorder for a particular state of less than normal consciousness (e.g., coma, near-coma, vegetative state, persistent vegetative state, minimally conscious state), and the like.

"Neurorehabilitation", as used herein, refers to any rehabilitation program that may be used for the purpose of improving, regaining, or restoring one or more neurological functions that may have been impaired (i.e., lost or diminished) in an individual as the result of a brain injury. Such neurorehabilitation programs comprise one or more neurostimuli designed to restore or improve one or more impaired neurological functions of the individual. Neurorehabilitation programs that may be used in conjunction with administering apomorphine as described herein include, without limitation, physical/sensory type stimulation protocols (exercises, tasks, light stimulation, voice stimulation, picture stimulation, tactile stimulation), electrical and/or magnetic stimulation regimens (e.g., electroconvulsive therapy, trans-cranial magnetic stimulation (TMS), deep brain stimulation (DBS); see, also, U.S. Patent No. 6,463,328), and/or drug-based stimulation regimens (e.g., using modafinil, caffeine, amphetamines). For example, a neurorehabilitation program may comprise having an individual who has sustained a brain injury perform or attempt to perform, often in multiple repetitions, one or more particular exercises or tasks designed to improve or restore one or more neurological functions. Thus, such exercises or tasks may include forms of physical therapy to promote development of an impaired motor function; exercises or tasks for improving aspects of cognitive functions as well, e.g., memory (as in a case of amnesia), reading, recognition of objects, comprehension and response to commands, and the like; and exercises or tasks designed to improve a combination of motor and cognitive functions; e.g., speech, writing, operating machines, etc. The goal of neurorehabilitation is to improve or restore one or more neurological functions that were impaired due to brain injury in an individual and, thereby, advance the individual toward increased participation and independence in self-care, mobility, and/or employment. It is understood that neurorehabilitation applied toan individual in an altered state of consciousness may be greatly limited to tasks that promote an emergence to a state of greater consciousness, e.g., response to simple commands, directed eye or body movement, response to various stimuli. According to the invention, in its simplest form, neurorehabilitation of an individual who has sustained a brain injury and who is in an altered consciousness state (e.g., coma, etc.; see discussion *infra*) is a method comprising administering to the individual apomorphine as described herein and monitoring the individual for emergence to a state of greater awareness. Upon emergence to a higher or normal (or pre-injury) state of consciousness, the individual may then be administered apomorphine as described herein in conjunction with a neurostimulation program or regimen, as described above, designed to enhance or restore one or more neurological function(s) that remain impaired after the individual has emerged to a greater or normal state of consciousness.

Any of a variety of disorders or conditions may lead to the impairment of one or more neurological functions of an individual. Traumatic brain injury (TBI) and stroke are among the most frequently occurring and widely known events that can cause brain injury and an associated impairment of one or more neurological functions. Among the variety of causes of TBI diagnosed each year in the United States and around the world are vehicle accidents, such as involving a car, motorcycle, or bicycle, in which an impact to the head causes loss of consciousness and coma. TBI patients may partially emerge from a coma to some higher state of consciousness (e.g., near-coma, vegetative state, minimally conscious state), rarely to the point of fully recovered state of awareness, or, even with emergence to full consciousness, rarely with a full array of normal neurological functions (e.g., normal communication ability, motor ability, memory, senses, etc.).

A variety of rehabilitation programs are currently in use to promote restoration of various neurological functions impaired by brain injury, e.g., for TBI and stroke patients. Neurorehabilitation treatments according to the invention may comprise administering to an individual who has sustained a traumatic brain injury apomorphine and one or more elements of a rehabilitation program currently used in the art to improve or regain neurological functions.

"Dopaminergic agent" or "dopaminergic compound" as used herein refers to a compound or composition that stimulates neurotransmission (signaling) through the dopaminergic system. Dopamine is the predominant catecholamine neurotransmitter in the mammalian brain. Neurotransmission through the dopaminergic system may occur by secretion of dopamine, inhibition of dopamine re-uptake, or by increasing synaptic concentrations of dopamine. Dopamine is involved in the control of a variety of neurological functions, including, but not limited to, cognition (e.g., consciousness memory), motor activity (e.g., movement), emotion, positive reinforcement, food intake, and neuroendocrine regulation (see, e.g. Missale et al., Physiol. Rev., 78: 189-225 (1998)). The dopaminergic system comprises at least five G protein-coupled dopamine receptor subtypes (D1 - D5) that are widely expressed on cells of the central nervous system and also in certain locations in the periphery, such as in kidney, vasculature, and pituitary (Id.).

Two major categories of dopaminergic agents are dopamine receptor agonists, which are compounds that bind dopamine receptors and stimulate neural signaling via the dopaminergic system, and dopamine precursors, which are compounds that are metabolized to the active neurotransmitter dopamine, which in turn bind to dopamine receptors to cause transmission of a neural signal. A variety of dopamine receptor agonists are known and include, without limitation, apomorphine, bromocriptine, amantadine, pergolide, pramipexole, ropinirole, fenoldopam, cabergoline, rotigotine, lysuride, talipexale, 7-OH DPAT, quinpirole, and SKF-38393 (Id.). Dopamine agonists have traditionally been further categorized as ergot derivatives (e.g., bromocriptine, pergolide, lysuride, cabergoline) and nonergot derivatives (e.g., ropinirole, pramipexaole) (see, e.g., Zafonte et al., J. Head Trauma Rehabil., 15: 1179-1182 (2000)).

The most widely used and best-known dopamine precursor is L-dopa (levodopa), which has been used in treating Parkinson's Disease, a disease characterized by dopamine depletion.

"Apomorphine" is a potent dopaminergic agent. It is a dopamine receptor agonist that binds directly to dopamine receptors (as opposed to metabolic agonists, such as L-dopa) and is reported to have a particularly high affinity for the D₂-like dopamine receptors (see, e.g., Missale et al., Physiol. Rev., 78: 189-225 (1998)). Apomorphine binds to both groups of dopamine receptors: the D₁-like group (D₁ and D₅) and the D₂-like group (D₂, D₃, D₄) of receptors. Prior therapeutic uses of apomorphine have included to induce vomiting (emetic agent), to reduce the number and severity of "off" phases in certain patients with Parkinson's Disease that are refractory to conventional dopaminergic (L-dopa) therapy, and to treat male impotence (see, e.g., U.S. Patent Nos. 6,306,437 and 6,436,950).

Considered as among the most potent of dopamine agonists in the arsenal of drugs for treating Parkinson's Disease, use of apomorphine to a Parkinson's Disease patient is recommended after a clinical finding that a patient has failed to respond to or must be removed from other more widely used agents for treating Parkinson's Disease, notably, L-dopa, bromocriptine, amantadine, or other agents (Colosimo et al., Clin. Neuropharmacol., 3: 243-259 (1994); Missale et al., Physiol. Rev., 78: 189-225 (1998)).

Apomorphine is chemically described as (R)-5,6,6a,7-tetrahydro-6-methyl-4H-dibenzo[de,g]quinolin-10,11-diol (molecular weight 267). The CAS registry number of apomorphine hydrochloride, anhydrous, is 41372-20-7. The chemical structure of apomorphine as a neutral compound can be represented by the following formula: 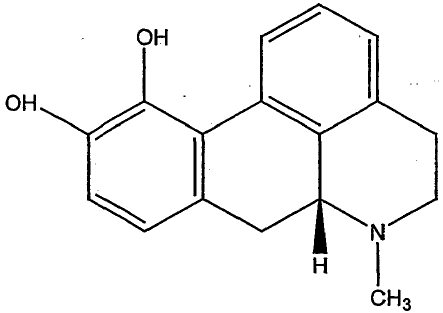 wherein only the stereochemically relevant hydrogen (at position 6a) is shown for structural clarity. The term "apomorphine", as used herein, encompasses not only a neutral free base form as in the above structure, but also individual stereoisomers and racemic mixtures thereof, pro-drug forms of apomorphine (i.e., compounds that are metabolized to apomorphine when administered to an individual), and any of a variety of salt forms of apomorphine, i.e., "acid addition salt" (or simply "acid salt") forms of apomorphine. The "acid" of an acid addition salt form of apomorphine may be inorganic (e.g., HCl) or organic (e.g., lactic acid, acetic acid). Preferably, apomorphine is used as the apomorphine hydrochloride salt. Such acid salt forms of apomorphine are particularly useful in pharmaceutically acceptable liquid (solutions, suspensions) compositions used to administer apomorphine to an individual. Use of obvious functional equivalents to the above structure, or chemically-modified equivalents of apomorphine, are also contemplated.

Apomorphine may be formulated for administration by a variety of routes, e.g., subcutaneously, sub-lingually (see, e.g., U.S. Patent Nos. 5,770,606; 6,306,437), and nasally (see, e.g., U.S. Patent No. 6,436,950). As discussed below, parenteral administration of apomorphine is particularly useful in methods of the invention, although other routes are not excluded. Apomorphine hydrochloride is generally the preferred, pharmaceutically acceptable, salt form employed for administering an effective amount of apomorphine to an individual according to the invention. Nevertheless, in addition to the hydrochloride salt form of apomorphine, other acid salt forms of apomorphine may be used in the invention including, but not limited to, a hydrobromide salt, a hydroiodide salt, a bisulfate salt, a phosphate salt, a lactate salt, a tartarate salt, a maleate salt, a succinate salt, a citrate salt, a gluconate salt, an acetate salt, and the like.

By "pharmaceutically acceptable" is meant a material that is not biologically, chemically, or in any other way, incompatible with body chemistry and metabolism and also does not adversely affect the desired, effective activity of a dopaminergic agent or any other component in a composition that may be administered to an individual to treat an impaired neurological function according to the invention.

"Consciousness" and "awareness" are, unless indicated otherwise, synonymous, and refer to the cognitive state of a person with respect to self and environment, consistent with usage by persons in the field of neurology. "Consciousness" has been technically defined as a spontaneously occurring state of awareness of self and environment comprising two dimensions, i.e., a "wakefulness dimension" (e.g., as evident by a circadian sleep cycle) and an "awareness dimension" (see, Ashwal, Brain & Develop., 25: 535-545 (2003)). Normal consciousness (full awareness) requires arousal, which is an independent, autonomic-vegetative brain function subserved by ascending stimuli, emanating from the pontine tegmentum, posterior hypothalamus, and thalamus that activate mechanisms inducing wakefulness. Cerebral cortical neurons and corresponding reciprocal projections to and from the major subcortical nuclei subserve awareness. It is understood that an individual may have a discernable circadian sleep-wake cycle, as in a vegetative or minimally conscious state, but lack a normal awareness of self and environment.

The terms "altered consciousness state disorder", "altered consciousness state", "ACS", "severely altered consciousness", "severe alteration in consciousness", "severely altered consciousness state", and "SACS", as used herein (whether or not written in upper or lower case letters), are synonymous and refer to the broad group of primarily cognitive neurological disorders of the brain that describe an impaired state of consciousness and include any degree of unconsciousness of an individual such that the individual is unable to be aroused to and/or to maintain a normal state of awareness of self and/or environment at a level that permits the individual, except for any physical disability, to care for himself, i.e., to function in a state of normal consciousness.

Altered state of consciousness disorders comprise those diagnosed according to established neurological standards and methods known in clinical neurology for assessing the conscious state of patients of brain injury. ACS disorders as understood herein include, but are not limited to, the following disorders, listed from lower to higher (i.e., more emergent) state of consciousness: coma, near-coma, vegetative state, persistent vegetative state (PVS), and minimally conscious state (MCS). It is further understood that ACS disorders of concern for the compositions for use described herein are those that are associated with a brain injury that is caused by the undesired detrimental or pathological event traumatic brain injury (TBI, e.g., head trauma from a fall or a vehicle accident). Compositions for use in the treatment of an ischemic event in an individual (blockage of normal blood flow anywhere in an individual resulting in brain injury, e.g., stroke), anoxic event (lack of oxygen to the brain), hypoxic oxygen event (lack of sufficient oxygen to the brain), drug-induced brain injury (e.g., alcoholic coma, heroin overdose, drug-associated locked-in disorder), and congenital or developmental brain disorders, such as lissencephaly) are also disclosed herein. ACS disorders treated according to the invention do not include states of consciousness as might result during hypnosis or some other form of subliminal suggestion as might be employed to achieve a desired behavioral modification.

Traumatic brain injury (TBI) and stroke are among the most frequent and widely known causes of brain injury that often result in an ACS disorder. TBI includes penetrating (e.g., gunshot wound) and non-penetrating (e.g., strike to the head) forms of brain trauma. Among the variety of causes for the millions of cases of TBI diagnosed each year in the United States and around the world, one of the most frequent is a vehicle accident, such as involving a car, motorcycle, bicycle, in which an impact to the head causes loss of consciousness and coma. Depending on the degree of trauma, such TBI patients may partially emerge from a coma to some higher state of consciousness (e.g., near-coma, vegetative state, minimally conscious state), but rarely to the point of fully recovered state of awareness, and rarely with a full array of normal neurological functions (e.g., normal communication ability, motor ability, memory, senses, etc.).

"Vegetative state" (VS) and "persistent vegetative state" (PVS) are usually distinguished in the art based on time course. Both VS and PVS are disorders in which the individual is considered to be in an unaware state that is, nevertheless, more emergent in awareness than coma, as evident by various characteristics such as eye opening or a discernable circadian sleep-wake cycle (see, e.g., Ashwal et al., Brain Develop., 25: 535-545 (2003)). PVS is typically the designation when the individual has been in VS for more than a week (see, e.g., The Multi-Society Task Force on Persistant Vegetative State, N. Eng. J. Med., 330): 1499-1508 (1994)).

"Minimally Conscious State" (MCS) is a relatively new designation for a defined altered consciousness state (see, e.g., Giacino et al., Am. Acad. Neurol., 58: 349-353 (2002); Ashwal et al., Brain Develop., 25: 535-545 (2003)). MCS is generally considered the most emergent of altered consciousness state disorders that currently may be assessed by clinical criteria (*Id.*).

"Coma" is a type of severely altered state of consciousness disorder characterized by a state of deep, unarousable (i.e., by normal stimuli), unresponsive, sustained, pathologic unconsciousness wherein the eyes are closed, and which results from dysfunction of the ascending reticular activating system either in the brainstem or in both the cerebral hemispheres (see, Ashwal, Brain & Develop., 25: 535-545 (2003)). Coma is understood to indicate the ACS disorder of least awareness and deepest state of unconsciousness, except for death, of an individual that has sustained a brain injury. Among notable characteristics, an individual in a coma does not have a discernable circadian sleep-wake cycle and lacks auditory, visual, communicative, and emotional functions (see, e.g., Giacino et al., Neurology, 58: 349-352 (2002)). Temporally, diagnosis of coma usually requires the period of unconsciousness to persist for at least one hour to distinguish coma from syncope, concussion, or other states of transient unconsciousness (*Id.*)*.* Individuals in a coma are unconscious because they lack both wakefulness and awareness dimensions (see, above).

"Near coma", as used herein, refers to a low state of consciousness that is, nevertheless, deemed more emergent in awareness than coma but less than vegetative state.

"Vegetative state", "VS", "persistent vegetative state", and "PVS", refer to the condition of complete unawareness of the self and the environment, but in contrast to coma, with sleep-wake cycles with complete or partial preservation of the hypothalamic and brain stem autonomic functions. Accordingly, a vegetative state is considered to be a more emergent state of consciousness than coma. The periods of wakefulness and sleeping of the vegetative state are typically irregular. In addition, when the eyes are opened, the individual fails to exhibit visual fixation or sustained visual tracking, and also may exhibit inconsistent head, trunk, and limb movements with respect to various stimuli (see, Ashwal, Brain & Develop., 25: 535-545 (2003)).

"Minimally Conscious State" or "MCS" is a severely altered state of consciousness disorder that has recently been defined as a more emergent state than vegetative state (see, Giacino et al., Neurology, 58: 349-353 (2002); Ashwal, Brain & Develop., 25: 535-545 (2003)). As a relatively newly defined state, not all practitioners have acceded to the legitimacy of or necessity for this category, opting for degrees within other older categories. Yet, the distinction from vegetative state is based on criteria that provide definite behavioral evidence of an awareness, albeit very limited, of self or environment based on one or more of four classes of behaviors, i.e., simple command-following, gestural or verbal "yes/no" response (regardless of accuracy), intelligible verbalization, and non-reflexive, "purposeful" behaviors (Ashwal, Brain Develop., 25: 535-545 (2003)). Functional interactive communication and use of extremities are considered key indications of further emergence from the minimally conscious state toward normal consciousness (*Id.*)*.* Whether or not the "minimally conscious state" *per se* is ultimately incorporated into the clinical diagnostic jargon of neurology does not, however, detract from or otherwise affect the methods and compositions described herein for treating impaired neurological function; changes in a state or pattern of consciousness and/or any other neurological function are readily assessed by any of a variety of methods and scales employed in clinical neurology (see, below).

Phrases that refer to administering or the administration of a drug, compound, or procedure "in conjunction with" apomorphine as described herein are understood to refer to any combination of therapeutic methods, compositions, or procedures that encompasses co-administration (i.e., together, e.g., as in a solution, dispersion, or other mixture), concurrent administration (essentially at the same time), and sequential administration (before or after) of a drug, other composition, or rehabilitative procedure (e.g., a task or exercise for cognitive and/or motor function), in addition to the administration of the apomorphine as described herein. It is also understood that administration of a drug or other composition "in conjunction with" apomorphine according to the invention may comprise using the same or different route used to administer apomorphine to an individual.

Terms such as "parenteral", "parenterally", and the like, refer to routes or modes of administration of a compound or composition to an individual other than along the alimentary canal. Examples of parenteral routes of administration include, without limitation, subcutaneous (s.c.), intravenous (i.v.), intramuscular (i.m.), intra-arterial (i.a.), intraperitoneal (i.p.), transdermal (absorption through the skin or dermal layer), nasal or pulmonary (e.g., by inhalation or nebulization for absorption through the respiratory mucosa or lungs), direct injections or infusions into body cavities or organs, as well as by implantation into the body or connection to the body of any of any a variety of drug delivery devices (e.g., implantation of a time-release composition, depot, or device that permits active or passive release of a compound or composition into the body).

The terms "enteral", "enterally", "oral", "orally", "non-parenteral", "non-parenterally", and the like, refer to administration of a compound or composition to an individual by a route or mode along the alimentary canal. Examples of enteral routes of administration include, without, limitation, swallowing solid or liquid forms, sub-lingual (absorption under the tongue), nasojejunal or gastrostomy tubes (into stomach), intraduodenal administration, as well as rectal administration (e.g., suppositories for release and absorption of a compound or composition by in the lower intestinal tract of the alimentary canal).

The meaning of other terms will be evident by the context of use and, unless otherwise indicated, are consistent with the meanings understood by those skilled in the fields of neurology and neurorehabilitation.

### Assessment of Neurological Health

The neurological health, including state of consciousness and other neurological functions, of an individual who has sustained a brain injury is typically assessed and/or monitored by a neurologist or other skilled healthcare professional that employs one or more recognized diagnostic protocols or scales known in the art. Routine neurological examination procedures are known in clinical neurology for assessing motor function (e.g., body movement), cognitive function (e.g., consciousness, thinking, reasoning, memory, circadian sleep-wake rhythm), and "complex" neurological functions having motor and cognitive functions (e.g., speech, writing, problem solving, operating machines). Among such protocols are various scales that comprise a defined set of parameters or tasks that are conducted or administered by a trained practitioner to assess an individual's state of consciousness. Such scales for consciousness are highly useful in diagnosing neurological health as an assessment of a pattern or state of consciousness of an individual is typically deduced based on assessment of a variety of neurological functions, including not only cognitive functions (e.g., memory, circadian rhythm), but also motor functions (e.g., body and eye movements) and more complex functions that involve cognitive and motor functions (e.g., speech, response to commands). Such scales are particularly effective for tracking emergence from a lower state of awareness to a higher, more emergent, state, e.g., coma to PVS to MCS to full consciousness. Moreover, some scales may also permit discernment of changes in a pattern of consciousness and neurological functions that may take place within a particular ACS disorder. Thus, current scale procedures may permit a skilled practitioner to discern even subtle improvements in neurological function in an individual treated according to the invention. For example, it may be desirable to determine that progress, albeit slow, is being made within a particular altered consciousness state, or that improvement is being made in one function, such as communication, even if another neurological function, such as motor function, may not have been fully restored. Thus, observing a change in even a pattern of consciousness may become so significant as to eventually indicate a change to a higher (or lower) state of consciousness.

Examples of well-known, validated scales for assessing consciousness and other neurological functions include, but are not limited to, the Glasgow Outcome Scale, the Extended Glasgow Outcome Scale, the Kennedy Johnson Scale, the Disability Rating Scale, the Coma-Near Coma Scale, the Ranchos Amigos Scale, and standard neurological assessment protocols that provide clinical impressions of change. It is not uncommon for an assessment of consciousness and neurological health to use more than one of these scales, depending on the nature of the brain injury.

The Kennedy Johnson Scale, also known as Coma Recovery Scale (Giacino et al., JFK Coma Recovery Scale and Coma Intervention Program Treatment Procedures, (Center for Head Injuries, JFK Johnson Rehabilitation Institute, Edison, New Jersey, 1992); Giacino et al., Arch. Phys. Med. Rehabil., 72: 897-901 (1991)), is a standardized method of assessment for grading the level of neurobehavioral responsiveness following severe brain injury. It is comprised of 25 items that assess the presence or absence of specific neurobehavioral signs that reflect the integrity of brain function. Responses are evaluated in the areas of arousal/attention, auditory function, visual function, motor function, motor/verbal ability, and communication. The Kennedy Johnson Scale yields six subscale scores and a total score. A total score between 0 and 14 is interpreted as coma or vegetative state and a score between 15 and 25 as emergent awareness. A disadvantage of this scale is that if a patient is unable to maintain arousal after stimulation, the assessment needs to be discontinued.

The Disability Rating Scale (DRS; Rappaport et al., Arch. Phys. Med. Rehabil., 63: 118-123 (1982)) was originally developed and tested with older juvenile and adult individuals with moderate and severe traumatic brain injury. This scale tracks an individual from coma to re-integration into the community. Various items in this scale address impairment, disability, and handicap. The DRS is a 31-point scale ranging from 0 (no disability) to 30 (death). Accordingly, the maximum score a living patient can obtain is 29 (extreme vegetative state) and 1 to 28 represent different grades of disability. A disadvantage of this scale is that it is relatively insensitive at the low end of the scale (i.e., mild traumatic brain injury). In particular, the scale does not have the ability to reflect very subtle, but sometimes significant, changes in an individual within a specific window of recovery.

The Coma/Near Coma (CNC) Scale essentially expands the levels of the Disability Rating Scale (DRS) that incorporate the vegetative and extreme vegetative categories (i.e., DRS scores between 21 and 29) (see, e.g., O'Dell et al., Neurorehabil., 6: 45-55 (1996); Pilon et al., Brain Injury, 10: 421-437 (1996); Rappaport et al., Arch. Phys. Med. Rehabil., 73: 628-634 (1992); Talbot et al., Brain Injury, 8: 689-699 (1994)). The CNC scale has five levels: no coma, near coma, moderate coma, marked coma, and extreme coma.

The Glasgow Outcome Scale (GOS; see, Jennet and Bond, Lancet, 1: 480-484 (1975)) is another widely used scale for assessing severe brain damage. A further modification of the Glasgow Outcome Scale, i.e., the Extended Glasgow Outcome (GOS-E) Scale (Wilson et al., J. Neurotrauma, 15: 573-85 (1998)), has also found use in assessing consciousness and other neurological functions in individuals having an ACS disorder.

Of course, the most desired outcome of the use of the treatment as described herein to an individual who has sustained a traumatic brain injury with an ACS disorder is the total emergence to normal functional awareness with restoration of all neurological functions at least to the level that existed prior to brain injury.

### Use of High Potency Dopaminergic Agents

Dopaminergic potency of a compound may be assessed on the basis of affinity for binding to one or more dopamine receptors or any assay that permits measurement of a compound's ability to stimulate signaling through the dopaminergic system (Missale et al., Physiol. Rev., 78: 189-225 (1998)). Dopaminergic potency may also be indicated by the effect a compound is observed to have on the brain, e.g., per unit dose.

This invention provides compositions for use for treating impaired neurological function in an individual who has sustained a traumatic brain injury comprising administering to the individual a high potency dopaminergic agent. Which is apomorphine. Apomorphine is classified as a highly potent dopamine agonist (Colosimo et al., Clin. Neuropharmacol., 3: 243-259 (1994)) and is, therefore, useful in the compositions for use in the invention. Prior therapeutic use of apomorphine as an emetic agent to induce vomiting is consistent with apomorphine's potent pharmacological effect upon the medullary chemoreceptors. More recently, apomorphine has been recommended for treating male impotency (see, e.g., U.S. Patent Nos. 6,306,437 and 6,436,950) and for dystonia (see, e.g., Colosimo et al., Clin. Neuropharmacol.,17: 243-259 (1994)). The most common use for apomorphine continues to be as a replacement for L-dopa in treating Parkinson's Disease or as a treatment for severe motor fluctuations in Parkinson's Disease patients who have undergone chronic L-dopa therapy (Colosimo et al., Clin. Neuropharmacol., 17: 243-259 (1994)).

In addition to its ability to bind the array of major dopamine receptors, apomorphine possesses pharmacological properties that distinguish this potent dopamine agonist over other compounds for use according to the invention. Of particular relevance here is the fact that apomorphine can be conveniently and easily prepared and administered to an individual by a parenteral route. Apomorphine has a low oral bioavailability because of extensive first pass hepatic metabolism, yet equilibrates quickly between the blood and tissues because of its high lipophilicity. In contrast to its poor bioavailability by oral administration, apomorphine is rapidly and completely absorbed when parenterally administered. Thus, apomorphine has a relatively quick onset of action when administered parenterally and is known to have a lower incidence of psychological effects than other dopaminergic agents (Lees et al., Fund. Clin. Pharmacol., 7(3-4): 121-128 (1993)).

Moreover, parenteral administration of apomorphine and, indeed, other dopamine agonists, is particularly well suited for treating a brain-injured individual who may not be fully conscious (e.g., in a coma or other ACS disorder), because parenteral administration typically does not require active participation or cooperation by the brain-injured individual. Even if partially or fully emerged to a normal state of consciousness, such an individual may still lack sufficient neurological function to easily receive oral medications without mechanical intervention (e.g., gastrostomy or nasojejunal tubes). Accordingly, the benefits of parenteral administration of a dopamine agonist, such as apomorphine, that can be so formulated are most preferred for treating brain-injured individuals. Parenteral administration of a dopamine agonist to a brain-injured individual may be conveniently, routinely, and accurately provided by a healthcare provider using any of a variety of clinical devices and methods, e.g., by using a syringe device for single or multiple injections or by using a pump or device that provides a continuous, controlled infusion of the drug into the brain-injured individual. If the brain-injured individual shows progress in regaining or restoring one or more impaired neurological functions (e.g., by emergence to a higher or, most preferably, normal or pre-injury state of consciousness), parenteral delivery of a dopamine agonist may be continued, even in conjunction with an increasingly demanding neurorehabilation program of cognitive and motor tasks or exercises. Routine clinical neurological assessments of the individual should be made by a trained healthcare provider in order to determine or conclude whether or not further administration of a dopamine agonist, with or without a continuing neurorehabilitation program, is likely to provide further progress in restoring an impaired neurological function. Such assessments for continuing, halting, or modifying a therapeutic regimen for brain-injured individuals are routinely performed by persons who are trained in clinical neurology and neurorehabilition.

As discussed in more detail below, apomorphine is preferably administered to an individual by a parenteral route, e.g., subcutaneously such as through the abdominal wall in an area of high capillary flow. When administered subcutaneously, the plasma half-lives of apomorphine are approximately 15 minutes and 70 minutes, fitting a two compartment model of biodistribution (Nicolle, Fund. Clin. Pharmacol., 7: 245-252 (1993)). The hydrochloride salt of apomorphine is a particularly useful form for preparing pharmaceutically acceptable solutions of apomorphine for parenteral administration to an individual according to the invention.

Apomorphine is rapidly and completely absorbed from subcutaneous tissues and is rapidly cleared. The effects of apomorphine are observed within five minutes following subcutaneous bolus administration. In the ACS patient, this rapid on and off allows better definition for a more exact onset of stimulation and duration of stimulation, which may be advantageous for many reasons, especially for inducing or maintaining a circadian rhythm in an ACS patient. Other types of agents having a longer half-life would continue to act on the CNS after termination of the infusion and would overlap with the sleep portion of the circadian cycle.

### Therapeutic Methods and Compositions

As there are no recognized animal models for coma or any other disorder of altered consciousness state, the use and outcome of therapeutic methods and compositions for treating impaired neurological function associated with brain injury, including those described herein, are typically based on actual clinical studies of human patients. Often such studies are able to assess one or no more than a few patients, who have sustained brain injuries. Accordingly, new therapeutic uses for regulated and approved drugs, such as apomorphine , develop slowly and with intense interest by practitioners in the fields of clinical neurology and neurorehabilitation. Such data are now emerging in support for the use of the compositions described herein (see, Examples, below).

This invention provides methods and compositions for use for treating an impaired neurological function in an individual who has sustained a traumatic brain injury comprising administering to the individual apomorphine.

A preferred dose of apomorphine that is administered to an individual is in the range of from 30 to 200 mg of apomorphine per day (mg/day) and, more preferably, in the range of from 48 to 128 mg/day. Daily dosing may be accomplished by single, multiple, or continuous injection or infusion of apomorphine into an individual. A preferred regimen for administering apomorphine to an individual according to the invention is to administer apomorphine at a rate of from 4 to 8 mg per hour for 12 to 16 hours. In a preferred aspect, apomorphine is adiministered in a manner to induce and mimic a normal circadian pattern in an individual in need of the same, the administration of apomorphine providing stimulation only during waking hours.

Commercially available preparations of apomorphine are typically provided at a concentration of 10 mg/ml. Subcutaneous administration of apomorphine at a concentration of 10 mg/ml is, however, not optimal as nodule formation (panniculitis) may occur at a site of injection. Accordingly, the concentration of a formulation of apomorphine administered by injection or infusion into an individual is preferably less than 10 mg/ml, e.g., 5 mg/ml.

Dosing for a particular individual (patient) who has sustained a traumatic brain injury will be determined by the attending neurologist or other skilled healthcare provider taking into account a variety of clinical parameters that characterize that patient, e.g., state of consciousness, overall neurological condition, other injuries, cardiovascular condition, age, gender, weight, possible genetic factors, and the like. It is also understood that persons skilled in the art are aware that doses of pharmacologically active compounds, such as apomorphine, may be expressed not only in terms of mass (e.g., mg) of drug administered per day, but other units as well as, including, but not limited to, mg per kilogram (kg) of body mass, mg per surface area, mg per unit volume of formulation, and the like. As used herein, discussion of dosages in terms of mg/day refer to mg per patient per day and are based on the commonly used standard of a 70 kg male human patient. Similarly, discussion of dosing in terms of mg of compound per kg of body weight (mass) assume a 70 kg male human being. Hence, it is understood that when treating an individual that is more or less than 70 kg a dose may be appropriately modified in accordance with standard pharmacological adjustments. Thus, various examples of doses described herein are readily converted by persons skilled in the art to various other dosing units (and vice versa) required for treating specific individuals with particular pharmaceutically acceptable formulations.

The present invention provides a method of making a medicament for treatment of impaired neurological function of an individual who has sustained a traumatic brain injury comprising use of apomorphine to prepare such medicament. The medicament is used according to the invention to treat impaired neurological function. In addition to conventional or traditional means for administering apomorphine to an individual (e.g., syringes, pumps, lozenges, pills), a number of recently developed or emerging technologies may also be employed in methods of treating impaired neurological function according to the invention. For example, various delayed or slow release solid formulations of a desired compound may provide delivery of the compound to an individual over a specified period of time. In addition to conventional electric or mechanical powered pumps, a continuous infusion of a compound to an individual may be accomplished by using an implantable passive or osmotic pump that contains a desired compound(s) and is swallowed by or implanted into an individual in order to release a defined amount of the compound(s) into the body of an individual. Still other drug delivery systems useful in the methods of the invention may comprise a molecule-based or nano-technology that permits one, few, or several individual molecules of a desired compound to be encapsulated or otherwise sequestered for release at a particular site and/or time after being implanted in, injected into, inhaled by, or ingested by an individual. An example of a molecule-based technology that may be employed to deliver compounds according to the invention are C60, C70, C76, and/or C80 buckministerfullerenes. Fullerenes or other molecular structures may effectively sequester (without limitation as to mechanism) one or several individual molecules of a desired compound and permit release of those molecules in an individual at a single or multiple times throughout a desired dosing period.

Neurorehabilitation according to the invention may comprise administering apomorphine to an individual who has sustained a traumatic brain injury in conjunction with a protocol or regimen of neurostimulation that is designed to restore or improve an impaired neurological function. Such protocols or regimens may include, without limitation, physical/sensory type protocols, electric and/or magnetic stimulation regimens, and/or drug-based stimulation regimens. Preferably, apomorphine is administered prior to or simultaneously with applying a program to the individual or with having the individual perform or attempt to perform an exercise or task designed for improving or restoring a neurological function of the individual. Physical/sensory stimulation programs or protocols are particularly useful in the invention and may include any of the well-known methods employed in clinical neurology and neurorehabilitation to stimulate a response of from one or more of the five senses. Such methods may include applying, without limitation, one or more sensory stimuli such as light, color, a visual scene (e.g., a picture), hot or cold temperature, tactile stimulation (e.g., for surface feeling), a smell, a taste, a sound (e.g., a voice of a family member), and the like.

In addition, various methods are now available that provide electric or magnetic stimulation to the brain. Such methods, which may be used in conjunction with administering apomorphine as described herein, include, but are not limited to, vagal nerve stimulation, cranial nerve stimulation by electrical pulse waveform, neuromodulation using a pulsed electrical stimulus, electroconvulsive therapy, trans-cranial magnetic stimulation (TMS), deep brain stimulation (DBS), and the like.

While not intending to be bound by any particular mechanism, the use of the compositions of the invention for treating impaired neurological function associated with a traumatic brain injury in an individual are intended to effectively and rapidly as possible increase neural transmission through the dopaminergic system to achieve one or more definite, observable, changes or endpoints, such as emergence from a lower to a higher state of consciousness, full arousal from unconsciousness to normal or pre-injury consciousness, and/or restoration of any other impaired neurological function associated with brain injury to the individual. In particular, the methods and compositions of the invention comprising apomorphine are not employed as chronic therapies as currently used to replace a progressive decline in the level of dopamine that characterizes neurodegenerative diseases, such as Parkinson's Diseases. Preferably, an apomorphine regimen as described herein is applied continuously to an individual for no longer than 18 to 24 months, more preferably no longer than 12 to 18 months, more preferably no longer than 6 to 12 months, and most preferably 6 to 24 weeks. The dopaminergic agent is administered within the parameters discussed herein until an improvement in a neurological function or return to a normal pre-injury state of conciousness is achieved. It is also possible that a healthcare provider may elect to apply methods and compositions as described herein more than once to a particular individual, e.g., after some hiatus from therapy.

Alternatively, the dosage regimen may be designed with a particular emergent outcome in mind. For example, an important feature of coma patients is the absence of any evidence of circadian rhythm. Restoration of circadian rhythm is also a desired initial endpoint in the recovery from coma. Consequently, it is desirable to arrange for the treatment regimen of coma patients to have a daily duration corresponding to the approximately 16 waking hours of a normal daily sleep/wake cycle. This, combined with the fact that coma patients are unable in many cases to take medication orally, makes it highly advantageous to prescribe continuous medication for approximately 16 hours to induce a circadian rhythm. A 12 to 16-hour dose regimen frequently leads to restoration of circadian rhythm in a coma patient.

Apomorphine described herein may advantageously also be administered to an individual in conjunction with any of a variety of other compounds that may provide one or more additional beneficial pharmacological activities. Such additional compounds may include, but are not limited to, an anti-emetic compound, another dopaminergic compound, an inhibitor of L-aromatic amino acid (L-dopa) decarboxylase activity, a catechol-O-methyltransferase (COMT) inhibitor, and combinations thereof. For example, some of the improvements made over the years for using L-dopa (levodopa) or other dopaminergic agents in chronic treatment regimens for Parkinson's Disease may also be applied in methods and compositions of the invention. Inhibitors of L-aromatic amino acid decarboxylase (also called L-dopa decarboxylase) and/or of catechol-O-methyltransferase (COMT) have been used to decrease degradation of extracerebral L-dopa. Thus, such inhibitors of enzyme activities that can degrade L-dopa may also provide an additional benefit of reducing acute side effects of L-dopa, such as nausea and vomiting. Examples of such useful inhibitors of L-aromatic amino acid decarboxylase activity include carbidopa and bensarazide. Useful COMT inhibitors include entacapone and tolcapone. Other anti-emetic agents that may be administered in conjunction with apomorphine according to the invention include, without limitation, prochlorperizine, trimethylbenzamide hydrochloride, chlormeprazine, prochlorpemazine, and combinations thereof.

As noted above, in addition to apomorphine, a variety of other dopamine agonists may also be included in the compositions described herein. Such dopamine agonists, include, e.g., bromocriptine, amantadine, pergolide, pramipexole, ropinirole, fenoldopam, cabergoline, rotigotine, lysuride, talipexale, 7-OH DPAT, quinpirole, and SKF-38393; all of which have been shown to exhibit a pharmacological profile for binding to one or more of the major dopamine receptors D₁, D₂, D₃, D₄, and/or D₅, to stimulate dopaminergic signaling (see, e.g., Missale et al., Physiol. Rev., 78: 189-225 (1998)). These dopamine agonists are less potent dopaminergic agents than apomorphine. The compositions and methods described herein comprising apomorphine may further comprise one or more of these other dopamine agonists, which may provide additional therapeutic activity, e.g., due to a different dopamine receptor binding profile, to enhance emergence toward full awareness and/or the restoration or improvement of neurological function(s) lost as the result of traumatic brain injury.

Use of compositions of the invention to treat an impaired neurological function associated with traumatic brain injury in an individual may also comprise administering to the individual apomorphine, as described herein, in conjunction with one or more central nervous system stimulants, such as, methylphenidate, pemoline, caffeine, amphetamines, modafinil, and combinations thereof.

The invention for use in treating impaired neurological function in an individual who has sustained a traumatic brain injury may also comprise administering a combination of apomorphine and L-dopa to the individual. In such cases, the L-dopa and apomorphine are typically administered by separate modes of administration, e.g., apomorphine, parenterally; L-dopa, orally. In addition, when administered in combination with apomorphine, L-dopa may be administered in a dose that is less than that used (i.e., < 1000 mg/day) for treatments of impaired neurological function using L-dopa as a monotherapy. Such combinations may provide an additive or synergistic clinical response.

The handling of various dosage forms and techniques for administering compounds for use according to the invention will be within the skill and knowledge of practitioners familiar with pharmaceutical formulations comprising dopaminergic agents and other compounds.

More generally, compositions useful in the invention may be formulated for administration to an individual according to standard pharmaceutical protocols and texts (e.g., Remington's Pharmaceutical Sciences, 18th ed., Alfonso R. Gennaro, ed. (Mack Publishing Co., Easton, PA 1990)). Thus, in addition to apomorphine, compositions useful in the invention may also comprise any of a number of various pharmaceutically acceptable buffers (carriers), excipients, or adjuvants that may provide one or more beneficial pharmacological properties, including but not limited to, more efficient or less painful administration to an individual, more efficient delivery of dopaminergic agent(s) to the central nervous system, and/or longer storage of composition (e.g., preservative to enhance shelf-life, reducing agents). Accordingly, pharmaceutical compositions of this invention may include, without limitation, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Apomorphine compositions containing reducing agents such as sodium metabisulfite, ascorbic acid, and sodium ascorbate are also particularly contemplated.

Compositions according to the invention may be in the form of a sterile injectable preparation, such as a sterile injectable aqueous solution or an oleaginous suspension. Suspensions may be formulated according to techniques known in the art using suitable dispersing or wetting agents (e.g., an anionic detergent). A sterile injectable preparation may also be a sterile injectable solution or suspension in a non-toxic, parenterally acceptable diluent or solvent, such as a solution in 1,3-butanediol. Pharmaceutically acceptable aqueous buffer solutions that may be employed for parenteral administration of a compound or composition described herein include, without limitation, sterile water, physiological saline, bacteriostatic saline (e.g., saline containing about 0.9% benzyl alcohol), phosphate-buffered saline, Hank's solution, Ringer's-lactate and the like. In addition, sterile, fixed oils have been conventionally employed as a solvent or suspending medium for use in administering compositions. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant.

The pharmaceutical compositions of this invention for oral administration may include, but are not limited to, capsules, tablets, caplets, pills, aqueous solution, oleaginous suspensions, syrups, or elixirs. In the case of tablets for oral use, carriers, which are commonly used include lactose and corn starch. Lubricating agents, such as magnesium stearate, may also be added. Capsules, tablets, pills, and caplets may also be formulated for delayed or sustained release. If desired, certain sweetening and/or flavoring and/or coloring agents may also be added.

For application topically, a composition of the invention may be formulated with a suitable ointment, gel, cream, or lotion containing the active components suspended or dissolved in a carrier. Carriers for topical administration include, but are not limited to, mineral oil, liquid petroleum, white petroleum, propylene glycol, polyoxyethylene polyoxypropylene compound, emulsifying wax and water. One or more emollients may be present to enhance penetration through the skin. Suitable carriers include, but are not limited to, mineral oil, sorbitan monostearate, polysorbate 60, cetyl esters wax, cetearyl alcohol, 2-octyldodecanol, benzyl alcohol and water. Topical administration may also be accomplished via transdermal patches or similar devices.

Compositions of this invention may also be administered in the form of suppositories for rectal administration. Such compositions can be prepared by mixing various desired pharmacologically active ingredients (e.g., dopaminergic agent(s), enzyme inhibitor(s), anti-emetic agent(s), etc.) with a suitable non-irritating excipient, which is solid at room temperature but liquid at body temperature and, therefore, will melt in the rectum space to release the active components that can be absorbed across the gut wall. Such materials include, but are not limited to, cocoa butter, beeswax and polyethylene glycols.

The pharmaceutical compositions of this invention may be administered nasally, in which case absorption of dopaminergic agent(s) may occur via the mucus membranes of the nose, or by inhalation or nebulization into the lungs (see, e.g., U.S. Patent No. 6,436,950). Such modes of administration typically require that the composition be provided in the form of a powder, solution, or liquid suspension, which is then mixed with a gas (e.g., air, oxygen, nitrogen, etc., or combinations thereof) so as to generate an aerosol or suspension of droplets or particles. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

Pharmaceutical compositions of the invention may be packaged in a variety of ways appropriate to the dosage form and mode of administration. These include but are not limited to vials, bottles, cans, packets, ampoules, cartons, flexible containers, inhalers, and nebulizers. Such compositions may be packaged for single or multiple administrations from the same container. Kits, of one or more doses, may be provided containing both the composition in dry powder or lyophilized form, as well an appropriate diluent, which are to be combined shortly before administration. The pharmaceutical composition may also be packaged in single use pre-filled syringes or in cartridges for use in auto-injectors, needleless jet injectors, and automatic pumps that can be attached to the individual. Other kits provided by the invention may comprise apomorphine in combination with an appropriate delivery system. Such delivery systems may include, without limitation, external pumps, implantable pumps, metered dosage delivery devices, and the like.

Various antimicrobial agents may also be used in compositions of the invention to prevent degradation and contamination. Such commonly used antimicrobial agents included phenol, benzyl alcohol, meta-cresol, methyl paraben, propyl paraben, benzalconium chloride, benzethonium chloride, and EDTA. Such agents are present at concentrations that will prevent the growth of bacteria, fungi, and the like, but be non-toxic when administered to the intended individual.

Consistent with good manufacturing practices, which are in current use in the pharmaceutical industry and which are well known to the skilled practitioner, all components contacting or comprising the pharmaceutical agent (dopaminergic agent) must be sterile and periodically tested for sterility in accordance with industry norms. Methods for sterilization include ultrafiltration, autoclaving, dry and wet heating, exposure to gases such as ethylene oxide, exposure to liquids, such as oxidizing agents, including sodium hypochlorite (bleach), exposure to high energy electromagnetic radiation, such as ultraviolet light, x-rays or gamma rays, and exposure to ionizing radiation. Choice of method of sterilization will be made by the skilled practitioner with the goal of effecting the most efficient sterilization that does not significantly alter a desired pharmacological activity of the dopaminergic agent and other components of a composition intended for administration to an individual. Ultrafiltration is a particularly useful method of sterilization for pharmaceutical compositions that are aqueous solutions or suspensions.

In order to more fully illustrate the invention, the following non-limiting example is provided.

### Examples

### Example Treatment by Subcutaneously Administered Apomorphine of Patients in Altered Consciousness State After Traumatic Brain Injury

Patients who have not fully regained consciousness following a severe TBI are treated as follows:
To be eligible for treatment, subjects meet the following inclusion criteria:
   1. Patients have remained in a persistent vegetative state (PVS) or in a minimally conscious state (MCS) for at least one month after injury
   2. Patients have reached a stabilized clinical state (e.g., afebrile, hemodynamic and internal balance stability)
   3. Patients will have a Disability Rating Scale (DRS) score between 21 and 29 (vegetative and extreme vegetative state) or patients will have a Coma Near-Coma (CNC) score of between 10 and 20 points (total items score).

Prior to initiation of apomorphine administration, ACS patients receive pretreatment with domperidone (or an equivalent anti-emetic agent, e.g., COMPAZINE® or TIGAN® anti-emetic agent) as a prophylactic against nausea and emesis over 24 to 48 hours prior to the first dose of apomorphine.

Apomorphine is administered subcutaneously via a needle connected to a specialized pump for at least 12 hours and no more than 16 hours a day. Apomorphine is prepared and administered in a solution at a concentration of 5-10 mg/ml. The initial dose of apomorphine on Day 1 is 2 mg/hour, and this is increased by 2 mg/hour daily, until the dose reaches 8 mg/hour on Day 4. If necessary, the dose of apomorphine is reduced, as indicated.

Treatment is initiated as soon as a patient is in a rehabilitation setting or the equivalent thereof and has had the anti-emetic pre-treatment. The daily dosing regimen is prescribed for 6 to 24 weeks, but is extendable to up to 2 years with observable improvement.

During exposure to apomorphine, all patients may receive a standardized program of sensory stimulation (or equivalent therapeutic regimen) once or twice a day for up to five days a week.

Following this treatment, the patients are expected to show improvement of coma score.

## Claims

1. Use of apomorphine for the manufacture of a medicament for the treatment of impaired neurological function resulting from a traumatic brain injury (TBI).

2. Use according to Claim 1, wherein said apomorphine is for parenteral administration or enteral administration along the alimentary canal.

3. Use according to Claim 2, wherein said parenteral administration is selected from the group of consisting of subcutaneous administration, intravenous administration, intramuscular administration, transdermal administration, nasal administration, and inhalation administration.

4. Use according to Claim 2, wherein said apomorphine is for parenteral administration in a single dose using a syringe device or in a continuous infusion using a pump.

5. Use according to Claim 2, wherein said enteral administration is selected from the group consisting of oral administration, sublingual administration, administration to the stomach by a tube, and rectal administration.

6. Use according to Claim 1, wherein the treatment further comprises administering an additional dopaminergic agent selected from the group consisting of L-dopa, bromocriptine, amantadine, pergolide, pramipexole, ropinirole, fenoldopam, cabergoline, rotigotine, lysuride, talipexale, 7-OH DPAT, quinpirole, SKF-38393, and combinations thereof.

7. Use according to Claim 6, wherein said additional dopaminergic agent is L-dopa.

8. Use according to Claim 7, wherein said L-dopa is administered at a dose that is less than 1000 mg/day.

9. Use according to Claim 1, wherein said apomorphine is a single stereoisomer.

10. Use according to Claim 1, wherein said apomorphine is a racemic mixture of stereoisomers.

11. Use according to Claim 1, wherein said apomorphine is an acid salt.

12. Use according to Claim 11, wherein said acid is selected from the group consisting of HCl, HBr, acetic acid, and lactic acid.

13. Use according to Claim 11, wherein said apomorphine is apomorphine hydrochloride.

14. Use according to Claim 1, wherein said apomorphine is for administration in an amount of 12 to 200 mg/day.

15. Use according to Claim 1, wherein said apomorphine is for administration in an amount of 48 to 128 mg/day.

16. Use according to Claim 14, wherein said amount of apomorphine is for administration over a period of 12 to 16 hours/day.

17. Use according to Claim 1, wherein said medicament is in the form of a kit comprising apomorphine in one or more containers adapted for use in a pump for continuous infusion of said apomorphine and instructions for administering apomorphine by continuous infusion with a pump.

18. Use according to Claim 17, wherein the kit further comprises said pump for administering said apomorphine in said one or more containers.

19. Use according to Claim 1, wherein said impaired neurological function is an impaired cognitive function, an impaired motor function, or a combination of impaired cognitive and motor functions.

20. Use according to Claim 1, wherein said impaired neurological function is an altered consciousness state (ACS) or amnesia.

21. Use according to Claim 20, wherein said altered consciousness state is an ACS disorder.

22. Use according to Claim 21, wherein said ACS disorder is selected from the group consisting of coma, near-coma, vegetative state, persistent vegetative state, and minimally conscious state.

23. Use according to Claim 21, wherein said treatment comprises administering said medicament in an amount and for a period sufficient to stimulate an improvement in a pattern of consciousness within an altered consciousness state or in a change from a lower to a higher state of consciousness.

24. Use according to Claim 23, wherein said improvement is indicated by improvement in a neurological function selected from the group consisting of circadian rhythm, eye opening, directed eye movement, directed body movement, response to verbal commands, communication ability, response to sensory stimulation, and combinations thereof.

25. Use according to Claim 23, wherein said improvement is a change from a lower to a higher state of consciousness.

26. Use according to Claim 25, wherein said higher state of consciousness is the state of full consciousness.

27. Use according to Claim 23, wherein said improvement in a pattern or state of consciousness is determined using a protocol selected from the group consisting of Glasgow Outcome Scale, Extended Glasgow Outcome Scale (GOS-E), the Kennedy Johnson Scale, the Disability Rating Scale, the Coma-Near Coma Scale, Ranchos Amigos Scale, clinical impressions of change, and combinations thereof

28. Use according to Claim 1, wherein said TBI is the result of a fall on a surface or a vehicle accident.

29. Use according to Claim 1, wherein the treatment comprises administering said medicament for a period sufficient to promote an improvement in the functional independence of the individual.

30. Use according to Claim 29, wherein said improvement in the functional independence of said individual is indicated by improved communication ability, improved motor ability, improved ability for daily self care, and combinations thereof.

31. Use according to Claim 1, wherein the treatment further comprises administering an additional dopaminergic agent.

32. Use according to Claim 31, wherein said additional dopaminergic agent is selected from the group consisting of dopamine agonist, dopamine transport inhibitor, dopamine metabolism inhibitor, dopamine precursor, and combinations thereof.

33. Use according to Claim 31, wherein said additional dopaminergic agent is capable of crossing the blood brain barrier.

34. Use according to Claim 31, wherein said additional dopaminergic agent is administered by a parenteral or an enteral route.

35. Use according to Claim 34, wherein said enteral route is via a nasojejunal tube or a gastrostomy tube.

36. Use according to Claim 1, wherein the treatment comprises administering said medicament in conjunction with an anti-emetic agent.

37. Use according to Claim 36, wherein said medicament is co-administered, concurrently administered, or sequentially administered with said anti-emetic agent.

38. Use according to Claim 37, wherein said medicament is sequentially administered after administration of said anti-emetic agent.

39. Use according to Claim 36, wherein said anti-emetic agent is selected from the group consisting of a peripheral dopamine antagonist, a phenothiazine agent, a benzamide agent, a serotonin antagonist, a histamine antagonist, a parasympathetic depressant, and a meclizine agent.

40. Use according to Claim 36, wherein said anti-emetic agent is selected from the group consisting of domperidone, prochlorperizine, trimethylbenzamide hydrochloride, chlormeprazine, prochlorpemazine, and combinations thereof.

41. Use according to Claim 40, wherein said anti-emetic agent is domperidone.

42. Use according to Claim 1, wherein the treatment comprises administering said medicament in conjunction with a central nervous system stimulant selected from the group consisting of pemoline, caffeine, amphetamines, modafinil, and combinations thereof.

43. Use according to Claim 1, wherein the treatment comprises administering said medicament in combination with applying to said individual at least one sensory stimulus.

44. Use according to Claim 43, wherein said sensory stimulus is selected from the group consisting of light, color, a visual scene, hot temperature, cold temperature, tactile stimulation, a smell, a taste, a sound, and combinations thereof.

45. Use according to Claim 1, wherein the treatment comprises administering said medicament in conjunction with a procedure to provide electric and/or magnetic stimulation to the brain, said procedure selected from the group consisting of vagal nerve stimulation, cranial nerve stimulation by electrical pulse waveform, neuromodulation using a pulsed electrical stimulus, electroconvulsive therapy, trans-cranial magnetic stimulation (TMS), deep brain stimulation (DBS), and combinations thereof.

46. Use according to Claim 1, wherein the treatment comprises administering said medicament in conjunction with having said individual perform or attempt to perform a task or exercise to restore an impaired neurological function.

## Patentansprüche

1. Verwendung von Apomorphin zur Herstellung eines Medikaments zur Behandlung einer beeinträchtigten neurologischen Funktion, die von einer traumatischen Hirnverletzung (TBI) resultiert.

2. Verwendung nach Anspruch 1, wobei das Apomorphin für eine parenterale Verabreichung oder eine enterale Verabreichung über den Verdauungstrakt ist.

3. Verwendung nach Anspruch 2, wobei die parenterale Verabreichung ausgewählt ist aus der Gruppe bestehend aus subkutaner Verabreichung, intravenöser Verabreichung, intramuskulärer Verabreichung, transdermaler Verabreichung, nasaler Verabreichung und Verabreichung über Inhalation.

4. Verwendung nach Anspruch 2, wobei das Apomorphin für eine parenterale Verabreichung in einer Einzeldosis unter Verwendung einer Spritze oder in einer kontinuierlichen Infusion unter Verwendung einer Pumpe ist.

5. Verwendung nach Anspruch 2, wobei die enterale Verabreichung ausgewählt ist aus der Gruppe bestehend aus oraler Verabreichung, sublingualer Verabreichung, Verabreichung in den Magen über eine Sonde und rektaler Verabreichung.

6. Verwendung nach Anspruch 1, wobei die Behandlung weiter die Verabreichung eines zusätzlichen dopaminergen Mittels umfasst, das ausgewählt ist aus der Gruppe bestehend aus L-Dopa, Bromocriptin, Amantadin, Pergolid, Pramipexol, Ropinirol, Fenoldopam, Cabergolin, Rotigotin, Lisurid, Talipexal, 7-OH DPAT, Quinpirol, SKF-38393 und Kombinationen davon.

7. Verendung nach Anspruch 6, wobei das zusätzliche dopaminerge Mittel L-Dopa ist.

8. Verwendung nach Anspruch 7, wobei das L-Dopa in einer Dosis verabreicht wird, die geringer als 1000 mg pro Tag ist.

9. Verwendung nach Anspruch 1, wobei das Apomorphin ein einziges Stereoisomer ist.

10. Verwendung nach Anspruch 1, wobei das Apomorphin eine racemische Mischung von Stereoisomeren ist.

11. Verwendung nach Anspruch 1, wobei das Apomorphin ein Salz einer Säure ist.

12. Verwendung nach Anspruch 11, wobei die Säure ausgewählt ist aus der Gruppe bestehend aus HCl, HBr, Essigsäure und Milchsäure.

13. Verwendung nach Anspruch 11, wobei das Apomorphin Apomorphin-Hydrochlorid ist.

14. Verwendung nach Anspruch 1, wobei das Apomorphin für eine Verabreichung in einer Menge von 12 bis 200 mg pro Tag ist.

15. Verwendung nach Anspruch 1, wobei das Apomorphin für eine Verabreichung in einer Menge von 48 bis 128 mg pro Tag ist.

16. Verwendung nach Anspruch 14, wobei die Menge an Apomorphin für eine Verabreichung über einen Zeitraum von 12 bis 16 Stunden pro Tag ist.

17. Verwendung nach Anspruch 1, wobei das Medikament in Form eines Sets ist, das Apomorphin in einem oder mehreren Behältern umfasst, das für die Verwendung in einer Pumpe für eine kontinuierliche Infusion des Apomorphins geeignet ist und Anweisungen zur Verabreichung von Apomorphin über eine kontinuierliche Infusion mit einer Pumpe.

18. Verwendung nach Anspruch 17, wobei das Set weiterhin die Pumpe für die Verabreichung des Apomorphins in dem einen oder mehreren Behältern umfasst.

19. Verwendung nach Anspruch 1 wobei die beeinträchtigte neurologische Funktion eine beeinträchtigte kognitive Funktion, eine beeinträchtigte Motorik oder eine Kombination von beeinträchtigter kognitiver Funktion und Motorik ist.

20. Verwendung nach Anspruch 1, wobei die beeinträchtigte neurologische Funktion ein veränderter Bewusstseinszustand (altered consciousness state, ACS) oder Amnesie ist.

21. Verwendung nach Anspruch 20, wobei der veränderte Bewusstseinszustand eine ACS Störung ist.

22. Verwendung nach Anspruch 21, wobei die ACS Störung ausgewählt ist aus der Gruppe bestehend aus Koma, Nahkoma (near-coma), Wachkoma (vegetative state), persistierendes Wachkoma (persistent vegetative state) und Zustand minimalen Bewusstseins.

23. Verwendung nach Anspruch 21, wobei die Behandlung die Verabreichung des Medikaments in einer Menge und für einen Zeitraum umfasst, die ausreichen, um eine Verbesserung in einem Muster des Bewusstseins innerhalb eines veränderten Bewusstseinszustands oder eine Änderung von einem niedrigeren zu einem höheren Zustand des Bewusstseins zu stimulieren.

24. Verwendung nach Anspruch 23, wobei die Verbesserung gezeigt wird durch Verbesserung einer neurologischen Funktion, die ausgewählt ist aus der Gruppe bestehend aus Biorhythmus, Öffnen der Augen, gezielte Bewegung der Augen, gezieltes Bewegen des Körpers, Reaktion auf verbale Anweisungen, Fähigkeit zur Kommunikation, Reaktion auf sensorische Stimulation und Kombinationen davon.

25. Verwendung nach Anspruch 23, wobei die Verbesserung eine Änderung von einem niedrigeren zu einem höheren Zustand des Bewusstseins ist.

26. Verwendung nach Anspruch 25, wobei der höhere Zustand des Bewusstseins der Zustand des vollen Bewusstseins ist.

27. Verwendung nach Anspruch 23, wobei die Verbesserung in einem Muster oder Zustand des Bewusstseins durch Verwendung eines Protokolls ermittelt wird, das ausgewählt ist aus der Gruppe bestehend aus Glasgow Outcome Scale, Extended Glasgow Outcome Scale (GOS-E), Kennedy Johnson Scale, Disability Rating Scale, Coma-Near Coma Scale, Ranchos Amigos Scale, klinische Eindrücke von Veränderungen und Kombinationen davon.

28. Verwendung nach Anspruch 1, wobei die TBI das Resultat eines Sturzes auf eine Oberfläche oder eines Fahrzeugunfalls ist.

29. Verwendung nach Anspruch 1, wobei Behandlung die Verabreichung des Medikaments für einen Zeitraum umfasst, der ausreicht, um eine Verbesserung in der funktionalen Unabhängigkeit des Individuums zu fördern.

30. Verwendung nach Anspruch 29, wobei die Verbesserung in der funktionalen Unabhängigkeit des Individuums durch verbesserte Fähigkeit zur Kommunikation, verbesserte motorische Fähigkeit, verbesserte Fähigkeit zur täglichen Selbstpflege und Kombinationen davon gezeigt wird.

31. Verwendung nach Anspruch 1, wobei die Behandlung weiter die Verabreichung eines zusätzlichen dopaminergen Mittels umfasst.

32. Verwendung nach Anspruch 31, wobei das zusätzliche dopaminerge Mittel ausgewählt ist aus der Gruppe bestehend aus Dopamin Agonist, Dopamintransport Inhibitor, Dopaminmetabolismus Inhibitor, Dopaminvorstufe und Kombinationen davon.

33. Verwendung nach Anspruch 31, wobei das zusätzliche dopaminerge Mittel in der Lage ist, die Blut-Hirn-Schranke zu überwinden.

34. Verwendung nach Anspruch 31, wobei das zusätzliche dopaminerge Mittel durch eine parenterale oder eine enterale Route verabreicht wird.

35. Verwendung nach Anspruch 34, wobei die enterale Route über eine nasojejunale Sonde oder eine Gastrostomiesonde ist.

36. Verwendung nach Anspruch 1, wobei die Behandlung die Verabreichung des Medikaments zusammen mit einem Antiemetikum umfasst.

37. Verwendung nach Anspruch 36, wobei das Medikament mit dem Antiemetikum zusammen verabreicht, gleichzeitig verabreicht oder sequenziell verabreicht wird.

38. Verwendung nach Anspruch 37, wobei das Medikament sequenziell nach der Verabreichung des Antiemetikums verabreicht wird.

39. Verwendung nach Anspruch 36, wobei das Antiemetikum ausgewählt ist aus der Gruppe bestehend aus einem peripheren Dopamin Antagonisten, einem Phenothiazin, einem Benzamid, einem Serotonin Antagonisten, einem Histamin Antagonisten, einem parasympatischen Beruhigungsmittel und Meclozin.

40. Verwendung nach Anspruch 36, wobei das Antiemetikum ausgewählt ist aus der Gruppe bestehend aus Domperidon, Prochlorperazin, Trimethylbenzamid Hydrochlorid, Chlormeprazin, Prochlorpemazin und Kombinationen davon.

41. Verwendung nach Anspruch 40, wobei das Antiemetikum Domperidon ist.

42. Verwendung nach Anspruch 1, wobei die Behandlung die Verabreichung des Medikaments zusammen mit einem Stimulans des zentralen Nervensystems umfasst, das ausgewählt ist aus der Gruppe bestehend aus Pemolin, Koffein, Amphetaminen, Modafinil und Kombinationen davon.

43. Verwendung nach Anspruch 1 wobei die Behandlung die Verabreichung des Medikaments in Kombination mit der Anwendung von mindestens einem Sinnesreiz an dem Individuum umfasst.

44. Verwendung nach Anspruch 43, wobei der Sinnesreiz ausgewählt ist aus der Gruppe bestehend aus Licht, Farbe, einer visuellen Szene, heißer Temperatur, kalter Temperatur, taktiler Stimulation, einem Geruch, einem Geschmack, einem Geräusch und Kombinationen davon.

45. Verwendung nach Anspruch 1, wobei die Behandlung die Verabreichung des Medikaments zusammen mit einem Verfahren umfasst, um elektrische und/oder magnetische Stimulation an das Hirn zu liefern, wobei das Verfahren ausgewählt ist aus der Gruppe bestehend aus Stimulation des Vagusnervs, Stimulation des Hirnnervs durch elektrischen Puls in Wellenform, Neuromodulation unter Verwendung eines gepulsten elektrischen Impulses, Elektrokrampftherapie, transkranielle Magnetstimulation (TMS), Tiefenhirn Stimulation (DBS) und Kombinationen davon.

46. Verwendung nach Anspruch 1, wobei die Behandlung die Verabreichung des Medikaments umfasst während das Individuum dazu gebracht wird, eine Aufgabe oder eine Übung auszuführen oder dies zu versuchen, um eine beeinträchtigte neurologische Funktion wiederherzustellen

## Revendications

1. Utilisation d'apomorphine pour la fabrication d'un médicament destiné au traitement d'une altération des fonctions neurologiques résultant d'une lésion cérébrale traumatique (LCT).

2. Utilisation selon la revendication 1, où ladite apomorphine est pour une administration parentérale ou une administration entérale le long du canal alimentaire.

3. Utilisation selon la revendication 2, où ladite administration parentérale est choisie dans le groupe consistant en l'administration sous-cutanée, l'administration intraveineuse, l'administration intramusculaire, l'administration transdermique, l'administration nasale, et l'administration par inhalation.

4. Utilisation selon la revendication 2, où ladite apomorphine est pour une administration parentérale en une dose unique en utilisant un dispositif de seringue ou dans une perfusion continue en utilisant une pompe.

5. Utilisation selon la revendication 2, où ladite administration entérale est choisie dans le groupe consistant en l'administration orale, l'administration sublinguale, l'administration à l'estomac par une sonde, et l'administration rectale.

6. Utilisation selon la revendication 1, où le traitement comprend en outre l'administration d'un agent dopaminergique supplémentaire choisi dans le groupe consistant en la L-dopa, la bromocriptine, l'amantadine, le pergolide, la pramipexole, le ropinirole, le fénoldopam, la cabergoline, la rotigotine, le lisuride, le talipexale, la 7-OH DPAT, le quinpirole, SKF-38393, et les combinaisons de ceux-ci.

7. Utilisation selon la revendication 6, où ledit agent dopaminergique supplémentaire est la L-dopa.

8. Utilisation selon la revendication 7, où ladite L-dopa est administrée à une dose qui est inférieure à 1000 mg/jour.

9. Utilisation selon la revendication 1, où ladite apomorphine est un stéréoisomère simple.

10. Utilisation selon la revendication 1, où ladite apomorphine est un mélange racémique de stéréoisomères.

11. Utilisation selon la revendication 1, où ladite apomorphine est un sel d'acide.

12. Utilisation selon la revendication 11, où ledit acide est choisi dans le groupe consistant en le HCl, le HBr, l'acide acétique, et l'acide lactique.

13. Utilisation selon la revendication 11, où ladite apomorphine est le chlorhydrate d'apomorphine.

14. Utilisation selon la revendication 1, où ladite apomorphine est pour une administration dans une quantité de 12 à 200 mg/jour.

15. Utilisation selon la revendication 1, où ladite apomorphine est pour une administration dans une quantité de 48 à 128 mg/jour.

16. Utilisation selon la revendication 14, où ladite quantité d'apomorphine est pour une administration sur une période de 12 à 16 heures/jour.

17. Utilisation selon la revendication 1, où ledit médicament est sous la forme d'un kit comprenant de l'apomorphine dans un ou plusieurs récipients adaptés à une utilisation dans une pompe pour une perfusion continue de ladite apomorphine et des instructions pour l'administration d'apomorphine par perfusion continue avec une pompe.

18. Utilisation selon la revendication 17, où le kit comprend en outre ladite pompe pour administrer ladite apomorphine dans lesdits un ou plusieurs récipients.

19. Utilisation selon la revendication 1, où ladite altération des fonctions neurologiques est une altération de la fonction cognitive, une altération de la fonction motrice, ou une combinaison d'une altération de la fonction cognitive et motrice.

20. Utilisation selon la revendication 1, où ladite altération des fonctions neurologiques est une altération de l'état de conscience (AEC) ou une amnésie.

21. Utilisation selon la revendication 20, où ladite altération de l'état de conscience est un trouble d'AEC.

22. Utilisation selon la revendication 21, où ledit trouble d'AEC est choisi dans le groupe consistant en le coma, un état proche du coma, un état végétatif, un état végétatif persistant, et un état de conscience minimale.

23. Utilisation selon la revendication 21, où ledit traitement comprend l'administration dudit médicament dans une quantité et pendant une période suffisante pour stimuler une amélioration d'un schéma de conscience au sein d'une altération de l'état de conscience ou d'un changement d'un état de conscience inférieur à supérieur.

24. Utilisation selon la revendication 23, où ladite amélioration est indiquée par une amélioration d'une fonction neurologique choisie dans le groupe consistant en le rythme circadien, l'ouverture des yeux, le mouvement dirigé des yeux, le mouvement dirigé du corps, la réponse à des commandes verbales, la capacité de communication, la réponse à une stimulation sensorielle, et les combinaisons de ceux-ci.

25. Utilisation selon la revendication 23, où ladite amélioration est un changement d'un état de conscience inférieur à supérieur.

26. Utilisation selon la revendication 25, où ledit état de conscience supérieur est l'état de pleine conscience.

27. Utilisation selon la revendication 23, où ladite amélioration d'un schéma ou d'un état de conscience est déterminée en utilisant un protocole choisi dans le groupe consistant en l'échelle de gravité de Glasgow, l'échelle de gravité de Glasgow étendue (GOS-E), l'échelle de Kennedy Johnson, l'échelle des taux d'invalidité, l'échelle du presque coma au coma, l'échelle de Ranchos Amigos, les impressions cliniques de changement, et les combinaisons de ceux-ci.

28. Utilisation selon la revendication 1, où ladite LCT est le résultat d'une chute sur une surface ou d'un accident de véhicule.

29. Utilisation selon la revendication 1, où le traitement comprend l'administration dudit médicament pendant une période suffisante pour favoriser une amélioration de l'indépendance fonctionnelle de l'individu.

30. Utilisation selon la revendication 29, où ladite amélioration de l'indépendance fonctionnelle dudit individu est indiquée par une amélioration de la capacité de communication, une amélioration de la capacité motrice, une amélioration de la capacité de prendre soin de soi quotidiennement, et les combinaisons de celles-ci.

31. Utilisation selon la revendication 1, où le traitement comprend en outre l'administration d'un agent dopaminergique supplémentaire.

32. Utilisation selon la revendication 31, où ledit agent dopaminergique supplémentaire est choisi dans le groupe consistant en un agoniste de la dopamine, un inhibiteur du transport de la dopamine, un inhibiteur du métabolisme de la dopamine, un précurseur de la dopamine, et les combinaisons de ceux-ci.

33. Utilisation selon la revendication 31, où ledit agent dopaminergique supplémentaire est capable de traverser la barrière hématoencéphalique.

34. Utilisation selon la revendication 31, où ledit agent dopaminergique supplémentaire est administré par une voie parentérale ou entérale.

35. Utilisation selon la revendication 34, où ladite voie entérale est réalisée par l'intermédiaire d'une sonde nasogastrique ou d'une sonde de gastrotomie.

36. Utilisation selon la revendication 1, où le traitement comprend l'administration dudit médicament conjointement avec un agent antiémétique.

37. Utilisation selon la revendication 36, où ledit médicament est coadministré, administré simultanément, ou administré séquentiellement avec ledit agent antiémétique.

38. Utilisation selon la revendication 37, où ledit médicament est administré séquentiellement après l'administration dudit agent antiémétique.

39. Utilisation selon la revendication 36, où ledit agent antiémétique est choisi dans le groupe consistant en un antagoniste périphérique de la dopamine, un agent de phénothiazine, un agent de benzamide, un antagoniste de la sérotonine, un antagoniste de l'histamine, un dépresseur parasympathique, et un agent de méclizine.

40. Utilisation selon la revendication 36, où ledit agent antiémétique est choisi dans le groupe consistant en la dompéridone, la prochlorpérazine, le chlorhydrate de triméthylbenzamide, la chlorméprazine, la prochlorpémazine, et les combinaisons de ceux-ci.

41. Utilisation selon la revendication 40, où ledit agent antiémétique est la dompéridone.

42. Utilisation selon la revendication 1, où le traitement comprend l'administration dudit médicament conjointement avec un stimulant du système nerveux central choisi dans le groupe constitué de la pémoline, la caféine, les amphétamines, le modafinil, et les combinaisons de ceux-ci.

43. Utilisation selon la revendication 1, où le traitement comprend l'administration dudit médicament en combinaison avec l'application audit individu d'au moins un stimulus sensoriel.

44. Utilisation selon la revendication 43, où ledit stimulus sensoriel est choisi dans le groupe consistant en la lumière, la couleur, une scène visuelle, une température très élevée, une température froide, une stimulation tactile, une odeur, une saveur, un son, et les combinaisons de ceux-ci.

45. Utilisation selon la revendication 1, où le traitement comprend l'administration dudit médicament conjointement avec une procédure visant à fournir une stimulation électrique et/ou magnétique au cerveau, ladite procédure étant choisie dans le groupe consistant en la stimulation du nerf vagal, la stimulation du nerf crânien par forme d'onde d'impulsion électrique, une neuromodulation en utilisant un stimulus électrique pulsé, l'électroconvulsivothérapie, la stimulation magnétique transcrânienne (TMS), la stimulation profonde du cerveau (DBS), et les combinaisons de ceux-ci.

46. Utilisation selon la revendication 1, où le traitement comprend l'administration dudit médicament conjointement avec l'entraînement dudit individu pour qu'il réalise ou tente de réaliser une tâche ou un exercice pour restaurer une altération des fonctions neurologiques.
